(19) Europäisches Patentamt
European Patent Office
Office européen des brevets

(11) Publication number: **0 241 309**
**A2**

(12) # EUROPEAN PATENT APPLICATION

(21) Application number: **87303166.0**

(22) Date of filing: **10.04.87**

(51) Int. Cl.⁴: **G 01 N 33/53**
G 01 N 27/00, C 12 Q 1/00,
C 12 M 1/40

(30) Priority: **10.04.86 GB 8608700**

(43) Date of publication of application:
**14.10.87 Bulletin 87/42**

(84) Designated Contracting States:
**CH DE FR GB IT LI**

(71) Applicant: **Genetics International, Inc.**
**128 Sidney Street**
**Cambridge Massachusetts 02139 (US)**

(72) Inventor: **Walton, Nicholas John**
**22 Summerfield**
**New Hinksey Oxford OX1 4RH (GB)**

**Chambers, Gill Alison**
**159 Flatford Place**
**Kidlington Oxford OX5 1TG (GB)**

(74) Representative: **Ruffles, Graham Keith et al**
**MARKS & CLERK 57-60 Lincoln's Inn Fields**
**London WC2A 3LS (GB)**

The application is published incomplete as filed (Article 93 (2) EPC) . The point in the description or the claim(s) at which the omission obviously occurs has been left blank. Page 15, line 16 ->.
A request for correction of page 15 of the description has been filed pursuant to Rule 88 EPC. A decision on the request will be taken during the proceedings before the Examining Division (Guidelines for Examination in the EPO, A-V, 2.2).

(54) **Measurement of electroactive species in solution.**

(57) In order to improve the sensitivity of electrochemical assays based on an electrochemical system capable of generating a current, where the system comprises as components an enzyme, a substrate, and a mediator, with the mediator mediating transfer of electrons between the enzyme and an electrode while the enzyme is catalysing a reaction of the substrate, the mediator is indirectly linked with the electrode by a compound capable of accumulating electrical charge for measurement after a delay period.

EP 0 241 309 A2

**Description**

MEASUREMENT OF ELECTROACTIVE SPECIES IN SOLUTION

Background of the Invention

This invention relates to assay techniques, and more especially to the measurement in solution, for instance either in a volume of liquid or in a moist environment upon a surface, of an electroactive species capable of transferring charge to or from an electrode.

There is a growing interest in the development of novel assay techniques which avoid the use of radioisotope labels. In particular, electrochemical methods have been recognised as providing a versatile detection system for use in an increasing number of assay formats. Such assays include amperometric measurement of solution electroactive species.

The present invention is particularly concerned with the measurement of very low concentrations of solution electroactive species, where the flux of electrons to or from an electrode surface is too low with normal experimental techniques to generate a measurable current over and above any background electrochemistry.

More particularly, the present invention relates to methods and equipment, involving measurement of electroactive species for detection, measurement or monitoring of components in complex mixtures, such as specific substrates within the mixture, specific enzymes, or components having a specific binding reaction such as an antibody-antigen reaction or such as the reaction of a DNA probe or the like with its target nucleic acid sequence.

The published European Patent Specification EP78636 (European Application No.82.305597) describes an assay method for a substrate of an enzyme. The assay is based on the direct electrochemical measurement of an enzyme-catalysed redox reaction utilising a mediator compound to transfer charge to or from an electrode in contact with the reaction. The reader is now referred to the EP78636, which is incorporated herein by reference.

By way of example, EP78636 explains the use of such mediator-linked measurements to detect glucose in whole blood. To achieve this end, a suitable system comprising a mediator and a glucose-catalysing enzyme (for example glucose oxidase or glucose dehydrogenase) are located on the surface of an electrode. The electrode is contacted with the whole blood, and the system reacts specifically with the glucose of that blood, giving rise to a current related to the glucose concentration. As mentioned in EP78636, a particularly suitable type of mediator is, for instance, a metallocene compound which has electronic properties rendering it capable of transferring an electron in a facile manner between the redox enzyme reaction and the electrode. Important examples of such metallocene compounds include ferrocene and various substituted ferrocene derivatives.

Subsequently, its was realized that the technology described in EP78636 might also be used to detect the presence or amount of an enzyme. In such a use, a ferrocene or other mediator is used with an excess amount of substrate in a wet or dry system to which an assay specimen can be added. The assay specimen will react with the system and generate a current only if a suitable substrate-reacting enzyme is present in the specimen. The system can be further elaborated to deal with a chain of enzyme reactions.

Typical examples of further electrochemical assay procedures and assay equipment are to be found in published EP127958 (European Application 84.303091), EP125867 (84.303085) EP125136 (84.303086) and EP125137 (84.303087), and the reader is now referred to these texts which are incorporated herein by reference.

At the heart of all these assays is the use of a mediator to transfer charge while the enzyme is catalysing a reaction of the substrate, and the detection of the consequential current which flows. Illustratively for an enzyme as in the glucose assay in EP78636, the assays rely fundamentally on the following cyclical sequences:

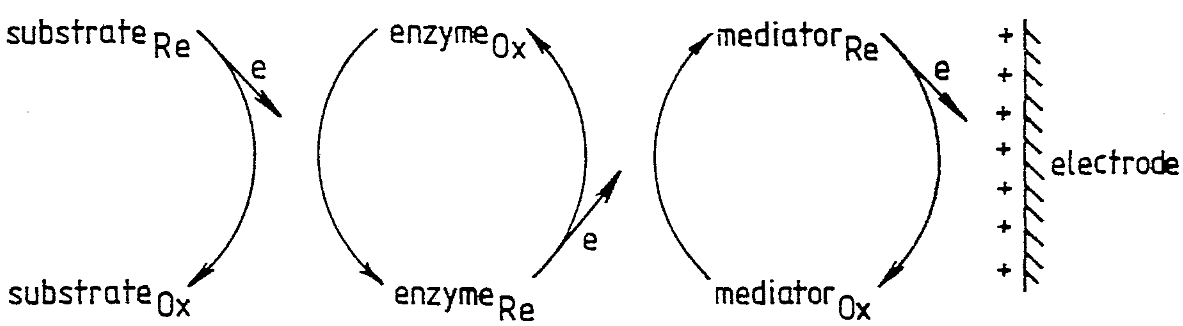


In this illustration the suffixes "Ox" and "Re" respectively indicate the oxidised and reduced forms of the substrate, enzyme or mediator, and "e" indicates an electron. It will be appreciated that the species "substrate$_{Ox}$" is more usually referred to as the product.

The net effect of the sequence is the continuing transfer of electrons to the electrode which is detected as a flowing current. The magnitude of the flowing current provides a quantitative measure for the assay. In this

respect, the reader is referred to Examples 7, 8 and 12 of EP78636, and the corresponding Figures 3, 6 and 7 which show the linear correlation between flowing current and glucose concentration.

Essentially, when a steady state enzyme reaction is mediated by a suitable amount of mediator, then a steady current is obtained. An important further development of the basic system is to be found in EP125139 (84.303090). The EP125139 describes the perturbation of the steady state system by effective variations in the amount of enzyme, substrate, mediator, or possibly electrode area. Such pertubations are brought about by a specific binding reaction, such as an antibody-antigen reaction or such as the reaction of a DNA (or like) probe with its target sequence. In this way, the EP125139 provides an electrochemical assay for a specific binding reaction.

In a preferred aspect, the EP125139 is concerned with an improvement in an immunoassay of the kind in which binding of an antigen to an antibody is assayed by detection of a label. The improvement consists in the steps of:

providing an electrochemical system capable of generating a current, the system comprising as components an enzyme, a substrate, and a mediator, with the mediator mediating transfer of electrons between the enzyme and an electrode while the enzyme is catalysing a reaction of the substrate;

employing one of the components as the label; and

detecting the specific binding as an alteration in the electrical current.

A typical and useful example of this improved immunoassay involves setting up a system containing the enzyme and substrate in a measurement system containing the necessary working electrode and any reference or counter electrode. There is also present in the system a supply of mediator which is conjugated chemically as a label to an antigen which is in turn linked by a specific binding reaction to an antibody. This further supply of conjugated mediator is unavailable for electrochemical use in mediation for as long as it remains linked with the large molecular antigen-antibody complex. Such a total system can be used for detection or assay of the antigen.

If a specimen containing the antigen is added to the system, some of the antigen will displace the mediator-labelled antigen. The displaced supply of mediator is then in a form conjugated only with the relatively small antigen molecule, and it can exert its mediating effect. The displaced mediator-labelled antigen can then transfer electrons between the enzyme and the electrode, thus producing a flowing current proportional to the amount of free antigen in the specimen.

## Objects of the Present Invention

The present invention is concerned with increasing the sensitivity of the various electrochemical assay systems described above. A specific object is an increase in the sensitivity of the systems involving specific binding reactions, whereby very low amounts of the electroactive species (the mediator) can be detected.

For example, the concentration range of many common therapeutic drugs and clinically significant analytes is often in the nanomolar or lower region. A particular object of this invention is a new assay method which enables detection in the low nanomolar concentration range.

## Summary of the present invention

In accordance with the present invention, the mediator is indirectly linked with the electrode by a material capable of accumulating electrical charge for measurement after a delay period.

In a general sense, the invention embraces improvements in the inventions of the European Patents cited above, where instead of direct mediation by the mediator between the redox enzyme and the electrode to give rise to a flowing current, the charge is accumulated on a compound which acts as a charge accumulator.

In the present system, a second redox species is used at open circuits as a reagent to accumulate charge during an incubation period from a first redox species acting as mediator. After acting upon the redox enzyme, the primary mediator is re-oxidised or re-reduced by the second redox species. The primary mediator thus cycles between the second redox species and the redox enzyme/substrate system, generating a pool of reduced or oxidised second redox species. This pool may then be electrochemically measured after a fixed time. The charge resulting from this step is thus the product of many primary mediator cycles, thus allowing small amounts of primary mediator to be measured as a current, potential or charge, and thus allowing assay of low levels of analyte.

The electrode can be interrogated after the fixed incubation time. For instance, such interrogation can be effected potentiometrically or galvanostatically, and in either case the electrode returned to its original potential with integration of the resulting current-time transient. The charge accumulated by the electrode and measured by the integration is proportional to the concentration of first redox species mediating the transfer of electrons between the electrode and the enzyme.

## Preferred Embodiments of the Present Invention

Any assay protocol can be employed, including for instance displacement or competitive procedures. The assay of this invention can be performed as a heterogenous or a homogenous assay. The assay is readily applicable to all kinds of analytes, including drugs, hormones, vitamins, food additives, etc.

The incubation time is best selected through routine experimentation, and typically will be from 10 seconds to 100 minutes, more typically 5 to 50 minutes and most typically 10 to 20 minutes.

The nature of the substrate and redox enzyme are not critical, and can be chosen for instance from the

examples of suitable substrate/enzyme pairings given in the European Patent Specifications mentioned above. By way of illustration, the substrate/enzyme pairing is preferably chosen from glucose/glucose oxidase; hydrogen peroxide/horse radish peroxidase; or other suitable well known pairings.

The nature of the mediator is also not critical, and can be chosen for instance from the examples of suitable mediators given in the European Patent Specifications mentioned above. By way of illustration, the mediator is preferably chosen from organic mediators, including ferrocene compounds; phenazines; carboranes; haloanils; etc.

In choosing the mediator, due regard is paid to the nature of the second redox species. By way of illustration, the second redox species is preferably chosen from poly(vinylferrocene) or prussian blue.

The second redox species, which acts as the charge accumulator, is not active as a mediator, and can be selected from known species. It can be non-mediating by virtue of physical and/or chemical proerties, taking in to account the reactivity, electrical charge, molecular size and other factors.

The second redox species can be non-mediating by immobilization, and then the assay of this invention will be heterogenous. The second redox species is preferably immobilized at the working electrode. Specific examples of redox-active electrode surface-modifications that can be tried include prussian blue (J Phys Chem (1983) 87, 105), copper hexacyanoferrate (III) (J Electrochem Soc (1983) 130, 396), nickel hexacyanoferrate (III) (J Electroanal Chem (1982) 137, 157), and poly(vinylferrocene) electrochemically precipitated onto glassy carbon electrodes (Polym Sci (1976) 14, 2433). A particularly preferred assay is based on the use of a poly(vinylferrocene)-modified electrode.

Thus, for a relatively straightforward heterogenous assay method for detecting redox-active species at the nanomolar concentration level, the method uses an electrode with the second redox species (the charge accumulator) immobilized on the electrode. The electrode is left at open circuit as a charge-accumulating device which, after potentiometric interrogation, is returned potentiostatically to its original potential. The charge passed in so doing is then equal to that acquired by the redox-modified electrode during the incubation period. This charge value is directly proportional to the concentration of redox-active mediator in the solution.

In a particularly preferred arrangement, all the components are on the electrode, allowing a dry strip assay similar to those described in EP127958. The dry strip electrode is preferably a carrier screen-printed with the active materials.

Schematically, and without being bound by theory, the present invention can be regarded as illustrated by the following scheme:

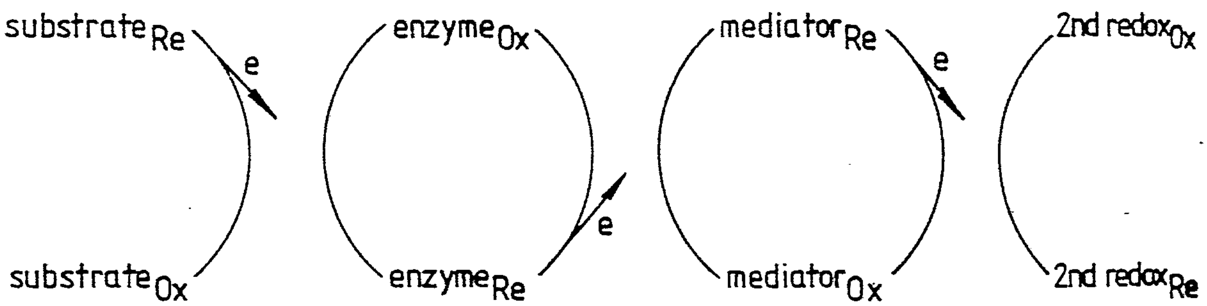


The charge accumulates on the second redox species. This charge accumulation occurs with continuing cycling of the mediator between oxidised and reduced forms as the enzyme catalyses oxidation of the substrate.

It is appropriate to note that in the present invention, the first redox species continues to act as a mediator during accumulation of charge on the second redox species. Thus, the mediator is continuously cycling between oxidized and reduced forms, as electrons are transferred between the redox species. No complex is formed between the redox species, and there is no similarity of the present invention with the assay of EP155135, where a charge-transfer complex is formed.

A protocol for a homogenous assay comprises admixing the substrate, first redox reagent, second redox reagent, and enzyme; poising the working electrode; incubating the system on open circuit; re-poising the working electrode; and monitoring the current.

In one preferred aspect, the invention consists in a method for detecting, measuring or monitoring the presence or amount of an electrochemically available mediator compound in a liquid medium, by contact with a redox enzyme acting upon a suitable substrate and thus providing charge for transfer by the mediator to or from an electrode of an electrical detection or measurement system: in which a second redox species, non-mediator to the enzyme, and present in large excess over the mediator, is also in contact with the electrode; and in which measurement of electrical charge, transferred from the mediator to the second redox species as the enzyme/substrate reaction proceeds, is made after a time interval sufficient to permit measurement of an accumulation of such charge and thus measurement of low levels of electrically available mediator activity.

Schematically, such an immunoassay can be illustrated by the following scheme:

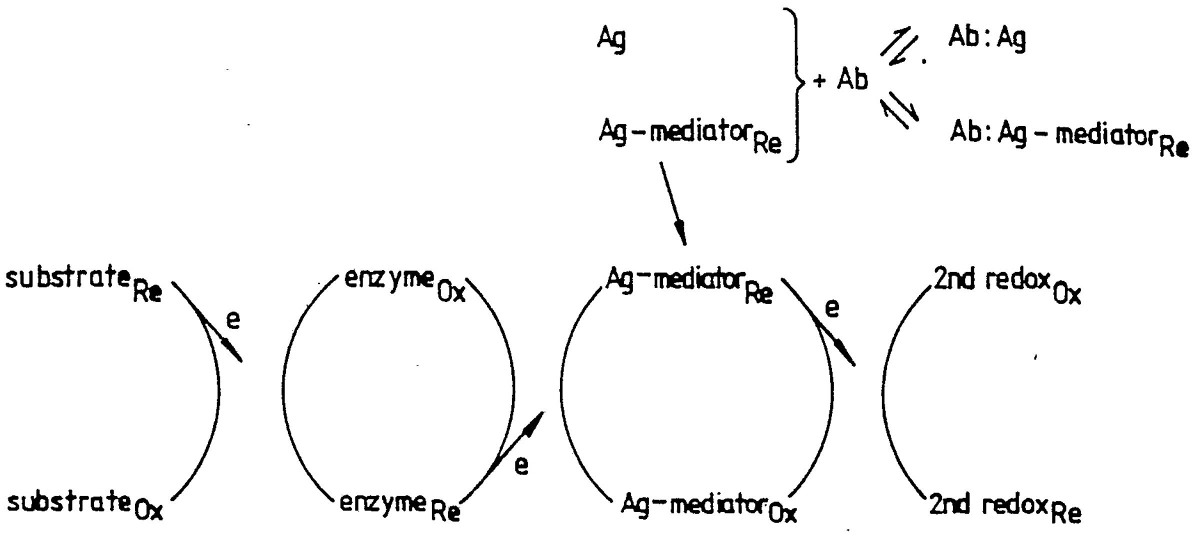


In this scheme, the antigen is shown "Ag", the antibody is "Ab" and the antigen-mediator conjugate is "Ag-mediator$_{Re}$" or "Ag-mediator$_{Ox}$". Use of a redox-labelled antigen (which acts as a mediator) in a competitive immunoassay system allows detection of unlabelled antigen.

A redox-modified electrode which is initially, say, fully oxidised can not be reduced at a finite rate by the solution amplification system, such as glucose oxidase/glucose, except in the presence of a solution small molecule redox mediator, "Ag-mediator". The rate at which the electrode modification will be reduced, or the extent to which it has been reduced after a fixed incubation time, will be in direct proportion to the solution concentration of active redox mediator. This mediator is the redox-labelled drug conjugate, of which there is a fixed quantity present. It is, however, in competitive equilibrium with unlabelled analyte, Ag, for a fixed number of solution antibody binding sites, Ab. When a redox-labelled conjugate is bound to antibody, forming "Ab:Ag-mediator", it is no longer able to mediate electron flow from glucose oxidase to the redox-modified electrode surface. Therefore the more unlabelled analyte there is present in the solution the more redox-labelled analyte there will be available for mediation and hence the more charge there will be accumulated by the redox-modified electron. This charge accumulated during a given incubation time can be then measured electrochemically by returning the electrode to its original start potential and integrating the current passed in so doing. The method relies on two reactions not taking place: (i) oxidation of reduced glucose oxidase by antibody-bound redox-labelled conjugate and (ii) oxidation of glucose oxidase directly by the redox-modified electrode in the absence of small molecule mediators. In practice, there is no difficulty in ensuring that these reactions do not proceed with appreciable rates.

The invention further provides an electrode system upon which is present one or more of the enzyme, substrate, mediator, and secondary redox material, together with an immunological or nucleic acid probe system. Such an electrode may be made up as a dry strip, typically containing a ferrocene-labelled antigen bound to its antibody, in addition to the enzyme/substrate system and the second redox species. Such an electrode, as part of a suitable measuring system known and described in the earlier European Patent Applications, can be used to detect the same antigen, which competes for the antibody and in so doing liberates the ferrocene-labelled antigen, which is then electrochemically available, in low concentrations. If desired, the electrode systems can be made up so as to contain only some of the species and so as to permit wet assay using different protocols.

The invention will be further described with reference to the following Examples and accompanying drawings.

Summary of the Drawings

Figure 1 is a calibration curve obtained in Example 2(ii).
Figure 2 is a calibration curve obtained in Example 2(iii).
Figure 3 is a calibration curve obtained in Example 2(iv).

Examples of the Invention

The electrochemical experiments were carried out at room temperature in a two compartment glass cell having a working volume of 1 $cm^3$. The working compartment accommodated a 1 $cm^2$ platinum gauze counter electrode in addition to the 5mm diameter glassy carbon working electrode. A saturated calomel reference electrode (SCE) was used in a side arm which connected to the working compartment via a Luggin capillary. A potentiostat, multimeter, and chart recorder were used to control the potential of the working electrode. Poised potentials were measured using a high input impedance ($> 2 \times 10^{14}\Omega$) programmable electrometer. All

assays were performed under oxygen-free argon. The charge accumulated during an assay was obtained as the digital integral of the current-time transient resulting when the electrode was scanned linearly with time (10 mV $s^{-1}$) back to the start potential of + 600mV: all potentials are with respect to a standard calomel electrode, "SCE". The transient data were acquired using a digital multimeter interfaced to a microcomputer.

Example 1

700 µl of TRIS buffer (0.1M, pH 7.14) containing ferricyanide ($Fe^{III}(CN)_6^{3-}$, 1 mMol $dm^{-3}$) and glucose (50mMol $dm^{-3}$) was placed in the sample side of the standard three-electrode electrochemical cell. To this system the required amount of ferrocene derivative was added ($E_{1/2}$ < +200 mV). Catalase (2500 U) and glucose oxidase (2000 U glucose oxidase, "GOD", E.C. 1.1.3.4 from Aspergillus niger, RMM 186,000) were then added. The solution was left in contact with a pyrolytic graphite working electrode on open circuit for 1 minute. The electrode was then poised at +400mV and the i/t transient was recorded. The current at 10 seconds was taken and a dose-response plot constructed. A linear response was obtained.

Example 2

Electrochemically Precipitated Poly(vinylferrocene): an Assay for Theophylline

Poly(vinylferrocene), "PVF", was prepared essentially according to the method of Smith et al. (Polym Sci (1976), 14. 2433). The polymer was electrochemically precipitated onto 0.2 $cm^2$ glassy carbon disc electrodes from non-aqueous solutions of $10^{-5}$M PVF in dichloromethane containing 0.1M tetra-n-butyl ammonium perchlorate (J Am Chem Soc (1978), 100, 3222). The deposition potential was typically +700mV and the immersion time 2 minutes. Upon removal of an electrode from the dichloromethane solution it was shaken to remove excess solution, sonicated in buffer (20mM potassium phosphate, pH 6.93 plus 0.1M sodium perchlorate) is order to remove any loosely held material, and then cycled linearly with time at a rate of 20mV $s^{-1}$ between -150 and +650mV in buffer until a steady voltammogram was obtained. All modified electrodes were then stored for a minimum of 12 hour in buffer at room temperature before being used.

The mediator was a ferrocene-ethanolamine of formula:

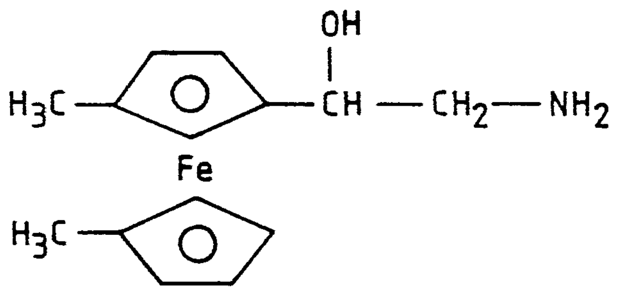

The redox-labelled conjugate was 8-ferrocenyl theophylline of formula:

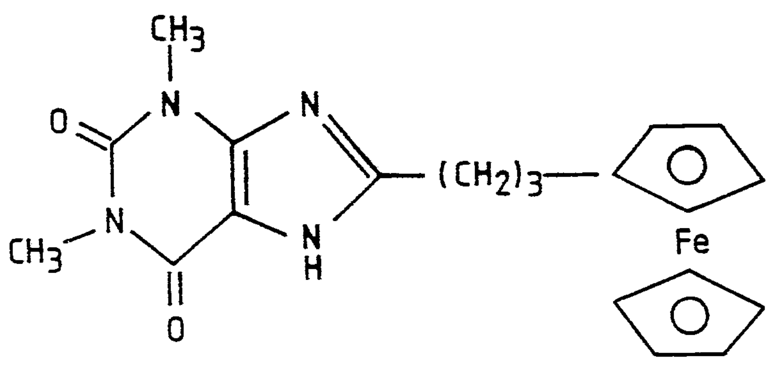

The antibody was anti-theophylline serum (Travenol anti-theo H113), and theophylline was the antigen. Standard ferrocene-theophylline conjugate solutions were prepared by dilution of a 1mM ethanol stock solution with buffer.

(i) For blank runs containing no mediator conjugate or antibody:

To 500 µl of 20mM potassium phosphate/0.1M sodium perchlorate buffer, pH 6.9, was added 50 µl of glucose solution (1.30M) followed by 50 µl of GOD solution (1.881mM). After thorough mixing, a PVF modified glassy carbon electrode was added to the cell, and potentiostatted at +600mV. At t = 0 minute, the cell was disconnected. After 10 minutes at open circuit the poised potential was measured, the electrode

re-potentiostatted at this potential and then scanned back to its original potential (+600mV), to give a current-time transient. From this transient the charge accumulated during the 10 minute incubation period was calculated. These blank runs gave the background response and acted as a control.

(ii) Runs containing varying amounts of mediator conjugate but no antibody:

To (550 - x) µl of buffer was added x µl of conjugate solution (4.228 x $10^{-7}$M), followed by 50 µl of glucose solution (1.30M) and 50 µl of GOD solution (1.881mM). After thorough mixing, a PVF-modified electrode was added to the cell and the assay continued as in (i).

The corrected charge values are plotted against concentration of redox-labelled theophylline conjugate in Figure 1.

(iii) Runs containing a fixed amount of conjugate (65nM) and varying amounts of antibody:

To (450 - y) µl of buffer as added 100µl of conjugate solution (4.228 x $10^{-7}$M) followed by y (in the range 15 to 120) µl of anti-theophylline serum (diluted 100-fold in buffer), and the solution incubated for 10 minutes at room temperature. After 10 minutes, 50 µl of glucose solution (1.30M) was added followed by 50 µl of glucose axidase solution (1.811mM), and the assay continued as in (i).

From the charge-accumulated results of experiments containing 65nM ferrocene-theophylline conjugate and then increasing volumes of anti-theophylline serum, it was found that the addition of antiserum resulted in a decrease in the amount of charge accumulated. The decrease is plotted in Figure 2 as a percentage of the charge accumulated in a standard experiment containing no added antiserum. The result is consistent with there being a reduction in the amount of redox-labelled conjugate available in solution for mediation as a result of some having become bound to the added antibodies. A 91% reduction in the charge normally accumulated in the presence of 65nM ferrocene-theophylline, glucose and glucose oxidase was observed upon addition of 120µl of antiserum. Addition of the undiluted anteserum (as supplied) resulted in complete inhibition of the catalytic current leading to film reduction with no measurable charge accumulation over and above the background response. Addition of antiserum, theophylline or a mixture of both to standard blank solutions containing just glucose and glucose oxidase has no measurable effect on the charge accumulated. The results obtained in each case coincided with the extrapolated background response between successive standard blank runs.

The results in Figure 2 were used to establish the assay conditions required for the detection of non-labelled theophylline.

(iv) Runs containing fixed amounts of both conjugate and antibody, but varying amounts of theophylline.

To (350-z) µl of buffer was added 100 µl of conjugate solution (4.228 x $10^{-7}$M) followed by z (in the range 100 to 350) µl of theophylline solution (1.237 µM). After thorough mixing, 100 µl of antitheophylline serum was added and the assay continued as in (iii).

Addition of non-labelled theophylline to runs containing 65nM ferrocene-labelled and a fixed amount of theophylline antiserum (100 µl) resulted in a propotional increase in the amount of charge accumulated compared to that accumulated in the presence of the conjugate and antiserum alone. This increase is due to the competitive equilibrium set up between the labelled and non-labelled theophylline for a fixed number of antibody binding sites. As the concentration of non-labelled theophylline is increased, so the proportion of ferrocene-labelled theophylline molecules bound to antibody is decreased. In other words, there is a proportional increase in the amount of redox-labelled conjugate in soltion available for mediation which results in a proportional increase in the amount of charge accumulated. It is this relationship between the charge accumulated and the concentration of added theophylline that forms the basis of the assay. Up to 72% reversal of inhibition was achieved upon addition of 666nM theophylline to experimental runs containing fixed amounts of conjugate (65nM) and antiserum (100 µl). This corresponded to a 10-fold excess of theophylline over the labelled theophylline which therefore has somewhat greater affinity for antibody binding sites compared with theophylline itself. By plotting the amount of charge accumulated during a series of experimental runs containing varying amounts of added theophylline it was possible to obtain the calibration curve as illustrated in Figure 3. These results show that the system in its present form is capable of reproducibly detecting theophylline at final concentrations within the range 50-700 nM, with an overall assay tine of 20 minutes.

## Example 3

### Electrodeposited prussian blue

A solution of ferrous sulphate ($Fe_2(SO_4)_3$, 20mMol $dm^{-3}$) and ferricyanide ($Fe^{III}(CN)_6^{3-}$, 20 mMol $dm^{-3}$) in 10 mM hydrochloric acid was placed in the sample cell of a three electrode electrochemical cell. A polished, sonicated gold working electrode was contacted with the solution and poised to +500 mV for 30 seconds. The electrode was then washed and cycled between 0 and +400 mV in 1M potassium chloride for 10 cycles. The modified electrode was removed and washed, giving a electrode ready for use in the assay. The modified electrode was placed in a phosphate buffer (20 mM, pH 6.9, 0.1 M KCl) containing glucose (10 mMol $dm^{-3}$) along with the primary redox active species, a ferrocene ($E_{1/2}$ < +200 mV). The electrode was poised as +400 mV and 100 U glucose oxidase added. The electrode was then disconnected and left to accumulate

charge for 10 minutes. After this time the potential of the electrode was measured on the high impedance voltmeter. The electrode was then poised to this measured potential and swept linearly with time back to +400 mV. The current-voltage transient was measured and the charge removed from the electrode coat was evaluated by integration of the area of the transient. A linear dose-response curve was again obtained.

Example 4

Printed Prussian Blue/Carbon on a Strip

Prussian blue was mixed with a carbon ink at 50 mg/g paste. This was then printed as the working electrode on a two-electrode strip as described in EP127958. Glucose oxidase (100 U) was placed on the surface of the working electrode, and the sample ferrocene in TRIS buffer (pH 7.2, 100 mMol $dm^{-3}$ glucose 0.15 M NaCl) was added. After 5 minutes the electrode was poised to +350 mV vs Ag/AgCl, and the current-time transient was recorded. The dose-response curve was linear.

The assay system as described herein is not only generally applicable for use with redox-labelled conjugates which shuttle charge from the enzyme to the second redox species but it can also be used "in reverse". For instance, a fully-reduced PVF-modified electrode could be used to reduce an enzyme system such as horse radish peroxidase plus hydrogen peroxide via the intermediacy of the redox-labelled conjugate.

## Claims

1. An electrochemical assay based on an electrochemical system capable of generating a current, potential or charge, the system comprising as components an enzyme, a substrate, and a mediator, with the mediator mediating transfer of electrons between the enzyme and an electrode while the enzyme is catalysing a reaction of the substrate, wherein the mediator is indirectly linked with the electrode by a compound capable of accumulating electrical charge for measurement after a delay period.

2. The assay of claim 1, wherein said electrical charge is accumulated during an incubation period, and said electrode is then interrogated.

3. The assay of claim 2, wherein for said interrogation, said electrode is returned potentiostatically or galvanostatically to its original potential with integration in either case of the resulting current-time transient.

4. The assay of claim 1, which is a heterogenous assay and said charge accumulator compound is immobilized at said electrode.

5. The assay of claim 1, which is a homogenous assay comprising admixing said substrate, a first redox reagent as said mediator, a second redox reagent as said charge accumulator compound, and said enzyme; poising said electrode; incubating the system on open circuit; re-poising said electrode; and monitoring the current.

6. The assay of claim 1 which is an immunoassay.

7. The assay of claim 6, where said mediator is a label on an antigen.

8. A dry strip electrode for use in the assay of claim 7 and comprising the enzyme, substrate, mediator-labelled antigen, charge accumulator compound, and antibody.

9. In an assay of the kind which involves providing an electrochemical system capable of generating a current, the system comprising as components an enzyme, a substrate, and a mediator, with the mediator mediating transfer of electrons between the enzyme and an electrode while the enzyme is catalysing a reaction of the substrate; the improvement which consists of the steps of providing a charge accumulator compound to accumulate a deficit or surfeit of said electrons during an incubation period, and subsequently monitoring the current represented by said deficit or surfeit.

0241309

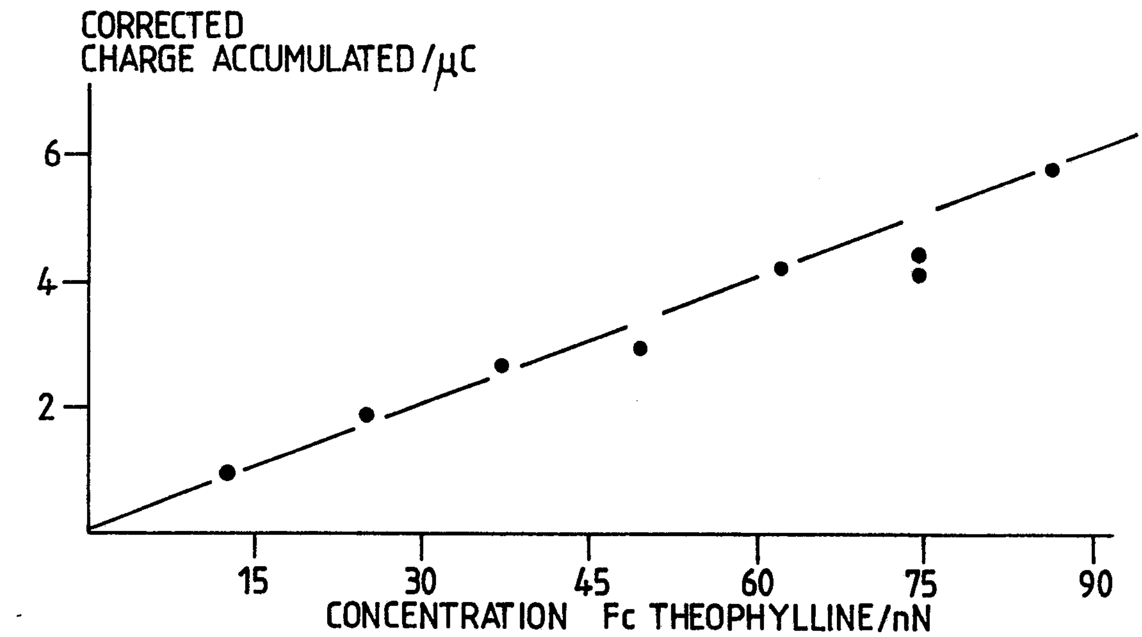


FIG.1.

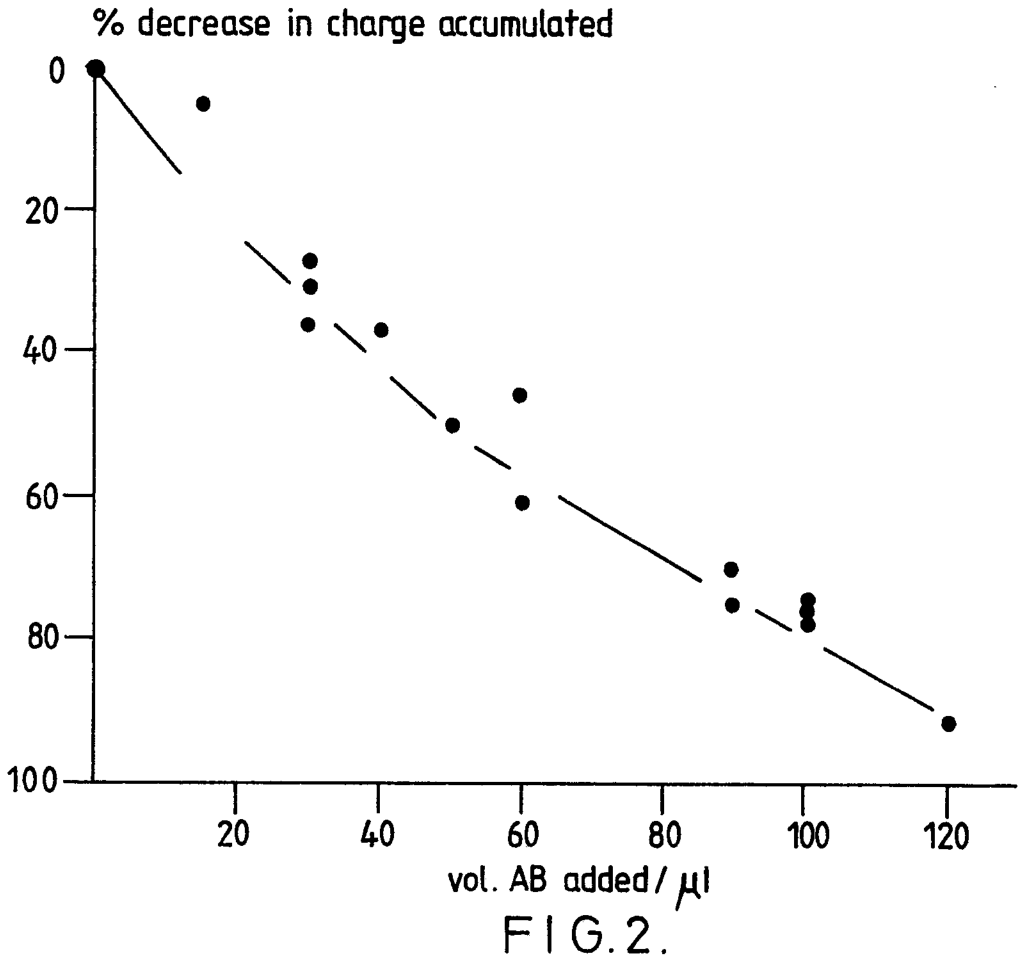


FIG.2.

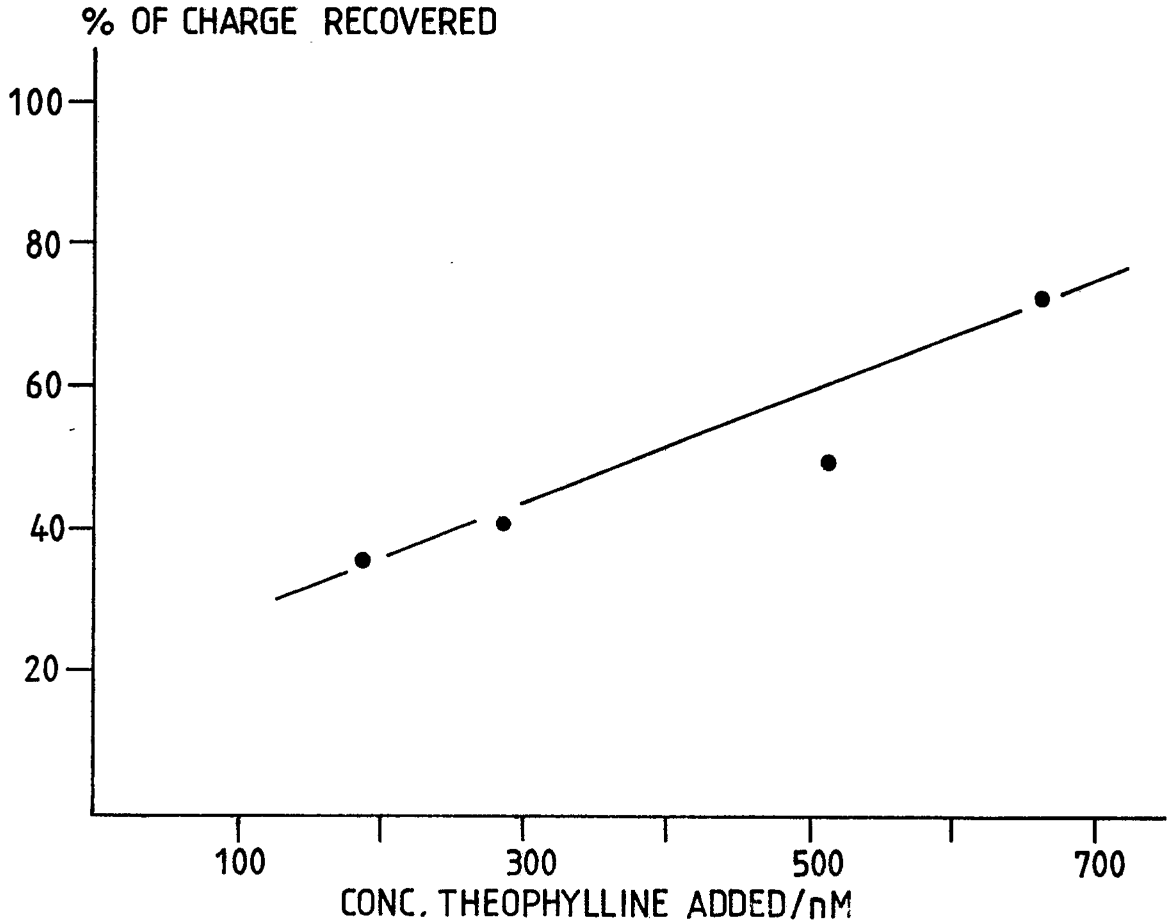
% OF CHARGE RECOVERED
100
80
60
40
20
100
300
500
700
CONC. THEOPHYLLINE ADDED/nM

FIG.3.